Europäisches Patentamt

European Patent Office

Office européen des brevets

(11) Veröffentlichungsnummer: **0 012 230 B1**

(12) **EUROPÄISCHE PATENTSCHRIFT**

(45) Veröffentlichungstag der Patentschrift:
**30.12.81**

(51) Int. Cl.³: **C 07 D 257/04**

(21) Anmeldenummer: **79104510.7**

(22) Anmeldetag: **15.11.79**

(54) Verfahren zur Herstellung von 1H-Tetrazol-1-Essigsäure und deren Estern.

(30) Priorität: **14.12.78 DE 2854015**

(43) Veröffentlichungstag der Anmeldung:
**25.06.80 Patentblatt 80/13**

(45) Bekanntmachung des Hinweises auf die Patenterteilung:
**30.12.81 Patentblatt 81/52**

(84) Benannte Vertragsstaaten:
**BE CH DE FR GB IT NL**

(56) Entgegenhaltungen:
**CH-A-570 989**
**CH-A-605 855**
**DD-A-110 272**
**DD-A-117 461**
**US-A-3 597 429**

(73) Patentinhaber: **DYNAMIT NOBEL AKTIENGESELLSCHAFT, Patentabteilung Postfach 1209, D-5210 Troisdorf, Bez. Köln (DE)**

(72) Erfinder: **Bison, Günter, Kleiststrasse 6, D-521 Troisdorf-Sieglar (DE)**
Erfinder: **Linkat, Norbert, Gartenstrasse 6, D-5205 St. Augustin 1 (DE)**
Erfinder: **Wolfes, Wolfgang, Dr., Bahnstrasse 11, D-5216 Niederkassel-Mondorf (DE)**

ACTORUM AG.

## Verfahren zur Herstellung von 1H-Tetrazol-1-Essigsäure
## und deren Estern

Das vorliegende Verfahren betrifft die verbesserte und vereinfachte Herstellung von Tetrazolessigsäure und deren Estern.

Es ist bekannt, dass zahlreiche Amin-Verbindungen mit Orthocarbonsäuren zu Iminoäthern umgesetzt werden können (De Wolfe, Carboxylic Ortho Acid Derivates, Academic Press (1970) Seiten 178/184).

Solche Iminoäther können nach Hagedorn et al (Berichte 99 (1960) S. 850/855) mit Stickstoffwasserstoffsäure oder deren Metallsalzen 1H-Tetrazol-Derivate bilden. Diese Reaktionen verlaufen keineswegs glatt, zumal die Iminoäther häufig in geringer Ausbeute entstehen und leicht zersetzlich sind.

Die Ausbeuten an Tetrazol-Derivaten sind daher durchweg gering.

Die DE-A-21 47 023 macht den Vorschlag, die Metallsalze der Stickstoffwasserstoffsäure, besonders Natriumazid, bereits bei der Bildung der Iminoäther in Gegenwart von insbesondere Essigsäure als Lösungsmittel zuzugeben, doch bleibt auch dieses Verfahren schwierig ausführbar und ergibt geringe Ausbeuten.

Stickstoffwasserstoffsäure ist bekanntlich hochexplosiv und erfordert selbst bei Verwendung in kleinsten Portionen besondere Schutzmassnahmen gegen Detonationen.

Bei dem Einsatz von Metallaziden wie z.B. dem nicht explosionsgefährlichen Natriumazid erfolgt eine Kondensation zum Tetrazolkörper nur dann, wenn in einem sauren Milieu gearbeitet wird.

So wird in Gegenwart von Essigsäure aus Metallaziden ständig entsprechend dem Massenwirkungsgesetz $HN_3$ freigesetzt, welche nach dem Verfahren der DE-A-21 47 023 als der eigentlich reagierende Stoff zu betrachten ist. Nach Beispiel 5 der genannten DE-A-21 47 023 wird z.B. im Gasraum eine $HN_3$-Konzentration von 16% gemessen, welche zum Durchdetonieren der Gasphase und damit des gesamten Ansatzes ausreichend ist.

Man kann jedoch nicht auf nicht-saure Lösungsmittel ausweichen, da beispielsweise bei Verwendung von Dimethylformamid die Ausbeute fast auf Null sinkt und auch die salzartig gebundene HCl eingesetzter Amin-Hydrochloride die Ausbeute nicht verbessert.

Demnach war der Nachteil der Detonationsfähigkeit bei der Herstellung offenbar nicht zu beseitigen, da $HN_3$ selbst offenbar die notwendige Reaktionskomponente bei der Entstehung des Tetrazolrings darstellt.

Es wurde nun gefunden, dass Tetrazol-Verbindungen ohne intermediäre Bildung von Stickstoffwasserstoffsäure mit hoher Ausbeute herstellbar sind.

Gegenstand der Erfindung ist ein Verfahren zur Herstellung von 1H-Tetrazol-1-essigsäure oder deren Estern durch Umsetzung eines Glycinestersalzes oder des Glycins, eines Orthoameisensäureesters und einer die Gruppierung $N_3$-enthaltenden Verbindung, welches dadurch gekennzeichnet ist, dass Trimethylsilylazid mit der Aminoverbindung und dem Orthoameisensäureester umgesetzt wird.

Es ist überraschend, dass die unpolare Verbindung Trimethylsilylazid den gewünschten Ringschluss bewirkt und zudem hohe Ausbeuten ermöglicht.

Es besteht der beträchtliche Vorteil, dass Trimethylsilylazid eine nicht-explosionsgefährliche Verbindung ist. Die Umsetzung von Trimethylsilylazid zu Tetrazol-Verbindungen verläuft ohne Freisetzung von Stickstoffwasserstoffsäure, selbst wenn saure Lösungsmittel wie Essigsäure oder Hydrochloride von Aminverbindungen anwesend sind. Bei der Umsetzung mit Trimethylsilylazid ist daher keine Detonation zu befürchten.

Auch die Herstellung von Trimethylsilylazid verläuft ohne Freisetzung von $HN_3$.

Bevorzugt ist die Herstellung von 1H-Tetrazol-1-essigsäurealkylestern mit 1 bis 8 Kohlenstoffatomen, besonders des Methylesters und Ethylesters. Die freie 1H-Tetrazol-1-essigsäure wird aus den Estern durch Verseifung erhalten. Die als Ausgangsstoffe eingesetzten Aminverbindungen und Orthoameisensäureester sind bekannt und können nach bekannten Methoden hergestellt werden.

Als Aminverbindungen für die Herstellung der Tetrazolessigsäure und ihrer Ester sind Glycin-Alkylester-Salze, insbesondere die Hydrochloride, gegebenenfalls auch die Glycinester selbst zu verwenden.

Als Orthoameisensäureester kommen insbesondere die Ethyl- und Methyl-Ester infrage.

Trimethylsilylazid kann nach der DE-A-19 65 741 einfach in etwa 98% der Ausbeute hergestellt werden.

Es ist möglich, zunächst durch Umsetzung der Amin-Verbindung und des Ortho-Esters den entsprechenden Iminoäther zu gewinnen und diesen anschliessend oder in gesonderter Reaktion mit Trimethylsilylazid umzusetzen. Entschieden bevorzugt ist jedoch, beide Reaktionen gleichzeitig durch Dosierung der Aminverbindung, des Orthoesters und des Trimethylsilylazids ablaufen zu lassen.

Dabei ist es von weiterem erheblichem Vorteil, die Herstellung des Trimethylsilylazids unmittelbar vor der Herstellung der Tetrazol-Verbindung vorzunehmen.

Die Molmengen von Glycinester und Trimethylsilylazid sollen im allgemeinen im stöchiometrischen Verhältnis stehen, jedoch sollte der Orthoester im Überschuss vorliegen, der bevorzugt mindestens 50 mol% bis zu 300 mol% beträgt.

Die Temperatur bei Ausführung des Verfahrens soll im allgemeinen zwischen 50 und 100 °C, besonders zwischen 65 und 80 °C liegen.

Die Reaktion wird im allgemeinen unter Eigendruck der Reaktionskomponenten im Gefäss mit aufgesetztem Rückflusskühler ausgeführt, jedoch kann die Reaktion auch im geschlossenen Gefäss unter Eigendruck stattfinden.

Die Ausbeute liegt im allgemeinen über 90%, vielfach im Bereich von 95%, bezogen auf Trimethylsilylazid. Es zeigt sich damit überraschend, dass auch die unpolare Verbindung Trimethylsilylazid, welche zur Bildung von freier $HN_3$ nicht befähigt ist, hohe Ausbeuten der Tetrazol-Verbindungen bewirkt, welche sogar bekannte Verfahren übertreffen.

Als Lösungsmittel werden bevorzugt aliphatische Carbonsäuren und insbesondere Essigsäure verwendet, obwohl auch in Sonderfällen weitere aus der genannten Literatur bekannte Lösungsmittel Verwendung finden können.

Unter den genannten Herstellungsbedingungen werden Tetrazolessigsäure und deren Ester, in sehr guten Reinheiten erhalten, welche mindestens 95% oder darüber betragen.

Herstellung von Trimethylsilylazid:
In einem 400-l-Emaillekessel mit Rührer, Rückflusskühler und Dosiervorrichtung werden 22,8 Liter (19,38 kg = 0,18 K mol) Trimethylchlorsilan eingefüllt, danach 640 g N-Methylpyrrolidon als Katalysator und 12,7 kg (0,19 K mol) $NaN_3$ hinzugefügt und das Gemisch innerhalb einer Stunde auf 50 °C erwärmt, wobei schwacher Rückfluss auftritt. Innerhalb weiterer zwei Stunden wird die Temperatur langsam bis auf einen Innentemperatur von 95 °C, d.h. den Siedepunkt von Trimethylsilylazid, gesteigert. Der Umsatz ist nach ca. 30 Minuten bei einer Innentemperatur von 95 °C unter Rückfluss ca. 98% (gaschromatographisch).

Beispiel 1
Der wie oben hergestellten Lösung aus Trimethylsilylazid werden nach Abkühlung auf ca. 40 °C 52,1 kg (0,49 K mol) Trimethylorthoformiat, 56,1 kg Eisessig, 14,6 kg wasserfreies Na-Acetat und 25 kg (0,18 K mol) Glycinethylesterhydrochlorid zugegeben. Das Gemisch wird unter Rühren langsam auf 70 °C erwärmt und 3 Stunden bei 70 °C gehalten. Nach Aufarbeitung durch Extraktion mit einem Lösungsmittel werden 27,02 kg 1H-Tetrazol-1-essigsäureethylester mit einer Reinheit von 95% gewonnen. Bei Raumtemperatur kristallisiert der anfangs ölige Ester völlig aus (Gesamtausbeute 96,2 Gew.-%, bezogen auf Trimethylsilylazid). Der erhaltene Ester kann ohne Nachbehandlung zur freien 1H-Tetrazol-1-essigsäure verseift werden.

Die abgetrennte Wasserphase ist frei vom Produkt und kann durch Verbrennen gefahrlos entsorgt werden.

Beispiel 2
Entsprechend Beispiel 1, jedoch bei einer Temperatur von 55 °C, wird in 5,5 Std. Reaktionszeit ein entsprechendes Ergebnis erhalten.

Beispiel 3
Entsprechend Beispiel 1, jedoch bei 100 °C, wird in ebenfalls guter Ausbeute und Reinheit der 1H-Tetrazol-1-essigsäureethylester erhalten.

Beispiel 4
Entsprechend Beispiel 1, jedoch unter Einsatz von 0,51 K mol Triäthylorthoformiat als Orthoester, wird der 1H-Tetrazol-1-essigsäureethylester mit entsprechenden Ergebnissen erhalten.

Beispiel 5
Entsprechend Beispiel 1, jedoch unter Einsatz der entsprechenden Molmenge Glycin-methylesterhydrochlorid wird der 1H-Tetrazol-1-essigsäuremethylester in ebenfalls über 90%iger Ausbeute erhalten.

**Patentansprüche**

1. Verfahren zur Herstellung von 1H-Tetrazol-1-essigsäure oder deren Estern durch Umsetzung eines Glycinestersalzes oder des Glycins, eines Orthoameisensäureesters und einer die Gruppierung $N_3$-enthaltenden Verbindung, dadurch gekennzeichnet, dass Trimethylsilylazid mit der Aminoverbindung und dem Orthoameisensäureester umgesetzt wird.

2. Verfahren zur Herstellung von 1H-Tetrazol-1-essigsäure oder deren Estern nach Anspruch 1, dadurch gekennzeichnet, dass Trimethylsilylazid unmittelbar vor der Herstellung der Tetrazol-Verbindung gebildet wird.

**Revendications**

1. Procédé d'obtention de l'acide 1H-tétrazol-1-acétique ou de ses esters par réaction d'un sel d'un ester de glycine ou de la glycine, d'un ester de l'acide orthoformique et d'un composé contenant le groupe $N_3$, caractérisé en ce qu'on fait réagir le triméthylsilylazide avec le composé amino et l'ester de l'acide orthoformique.

2. Procédé d'obtention de l'acide 1H-tétrazol-1-acétique ou de ses esters selon la revendication 1, caractérisé en ce que le triméthylsilylazide est formé directement avant la préparation du composé de tétrazole.

**Claims**

1. Process for the production of 1H-tetrazole-1-acetic acid or esters thereof by reaction of a glycine ester salt or of glycine, an orthoformic ester and a compound containing the grouping $N_3$, characterised in that trimethylsilylazide ist reacted with the amino compound and the orthoformic acid ester.

2. Process for the production of 1H-tetrazole-1-acetic acid or esters thereof according to claim 1, characterised in that the trimethylsilylazide is formed immediately before the production of the tetrazole compound.